# EUROPEAN PATENT SPECIFICATION

(11) **EP 4 260 924 B1**
(45) Date of publication and mention of the grant of the patent: **14.10.2026**
(21) Application number: 23167102.5
(22) Date of filing: 06.04.2023
(51) Int. Cl.: B01D 15/22, B01D 15/32, B01D 15/36, C12N 15/10, G01N 30/60

(54) **CHROMATOGRAPHY DEVICE AND METHOD OF USE**
CHROMATOGRAPHIEVORRICHTUNG UND VERFAHREN ZUR VERWENDUNG
DISPOSITIF DE CHROMATOGRAPHIE ET PROCÉDÉ D'UTILISATION

(30) Priority: 15.04.2022 US 202217721451
(43) Date of publication of application: 18.10.2023
(73) Proprietor: Cytiva US LLC, Marlborough, MA 01752 (US)
(72) Inventor: KAVARA, Aydin, Westborough, MA 01581 (US)
(74) Representative: Spjuth, Nora Sofia

(56) References cited:
- EP-A1- 1 321 176
- WO-A1-2016/093926
- US-A1- 2001 025 818

## Description

### BACKGROUND OF THE INVENTION

RNA (e.g., lentivirus RNA) and DNA (e.g., plasmid DNA) purification typically requires the use of different chromatography devices and dilution of high salt into low conductivity buffer.

There is a need for improved chromatography devices and methods of purification.

The present invention provides for ameliorating at least some of the disadvantages of the prior art. These and other advantages of the present invention will be apparent from the description as set forth below.

EP 1321176 A1 relates to a process and chromatographic device for isolating and purifying a polynucleotide in general, in particular plasmid DNA. WO 2016/093926 A1 discloses mixed bed ion exchange adsorbers comprising fibers that are compressed into a packed bed of fibers for purification of monoclonal antibodies.

### BRIEF SUMMARY OF THE INVENTION

An aspect of the invention provides a chromatography device comprising (a) a filter housing having an inlet and an outlet and defining a fluid flow path between the inlet and the outlet; (b) a porous filter arranged in the filter housing across the fluid flow path, the porous filter comprising (i) a first porous filter element; and (ii) a second porous filter element in contact with the first porous filter element, the second porous filter element arranged downstream of the first porous filter element, wherein the first porous filter element comprises at least one anionic exchange (AEX) membrane, and the second porous filter element comprises at least one hydrophobic interaction (HIC) membrane.

Another aspect of the invention provides a method of purification of enveloped viruses, such as lentivirus, the method comprising passing a fluid comprising enveloped viruses through a chromatography device comprising (a) a filter housing having an inlet and an outlet and defining a fluid flow path between the inlet and the outlet; (b) a porous filter arranged in the filter housing across the fluid flow path, the porous filter comprising (i) a first porous filter element; and (ii) a second porous filter element in contact with the first porous filter element, the second porous filter element arranged downstream of the first porous filter element, wherein the first porous filter element comprises at least one anionic exchange (AEX) membrane, and the second porous filter element comprises at least one hydrophobic interaction (HIC) membrane; binding the enveloped virus to the at least one AEX membrane; eluting the enveloped viruses from the at least one AEX membrane in high salt; binding the enveloped viruses to the at least one HIC membrane; and eluting the enveloped viruses in low salt buffer.

### BRIEF DESCRIPTION OF THE SEVERAL VIEWS OF THE DRAWING(S)

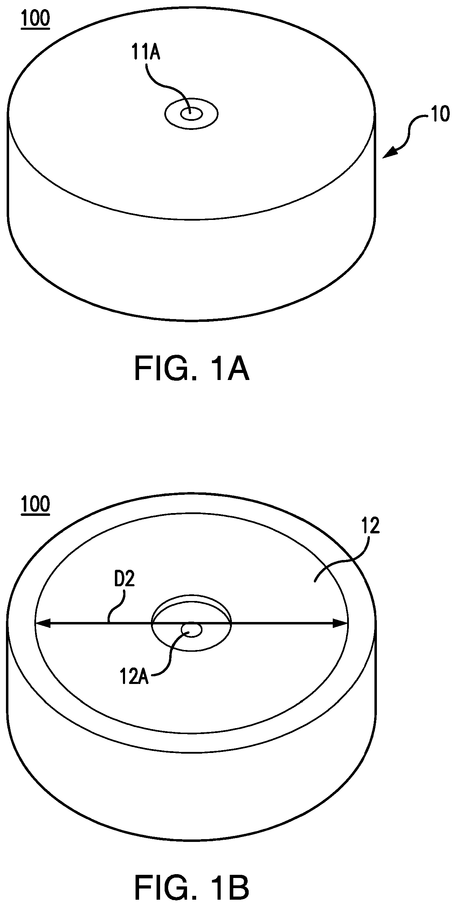

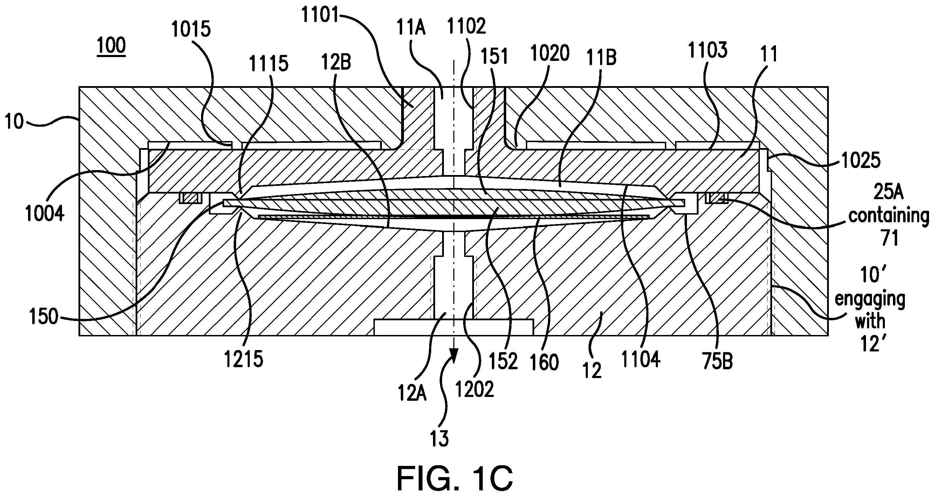

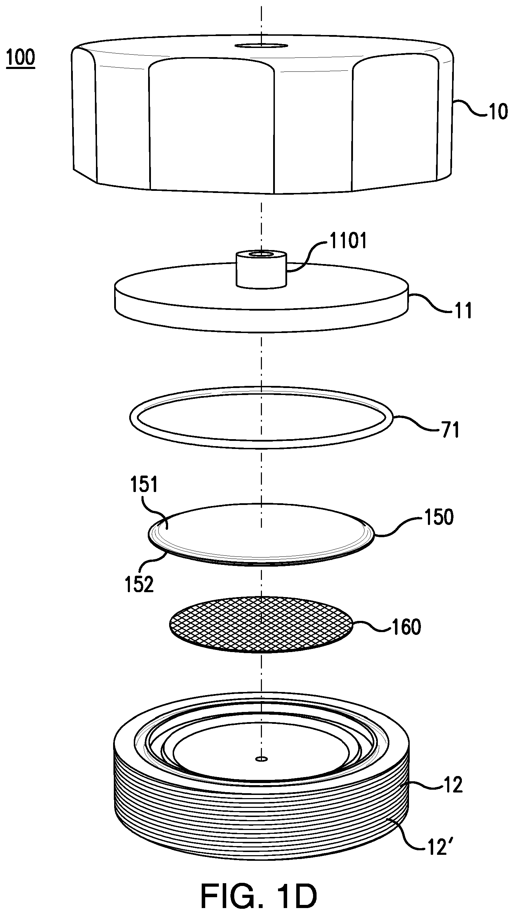

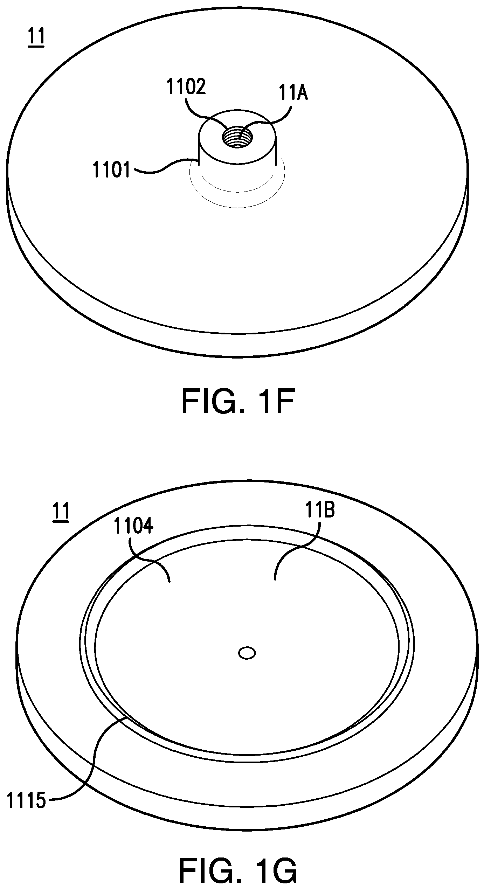

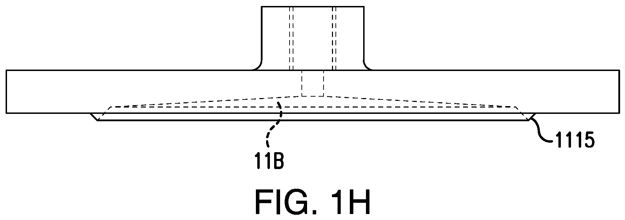

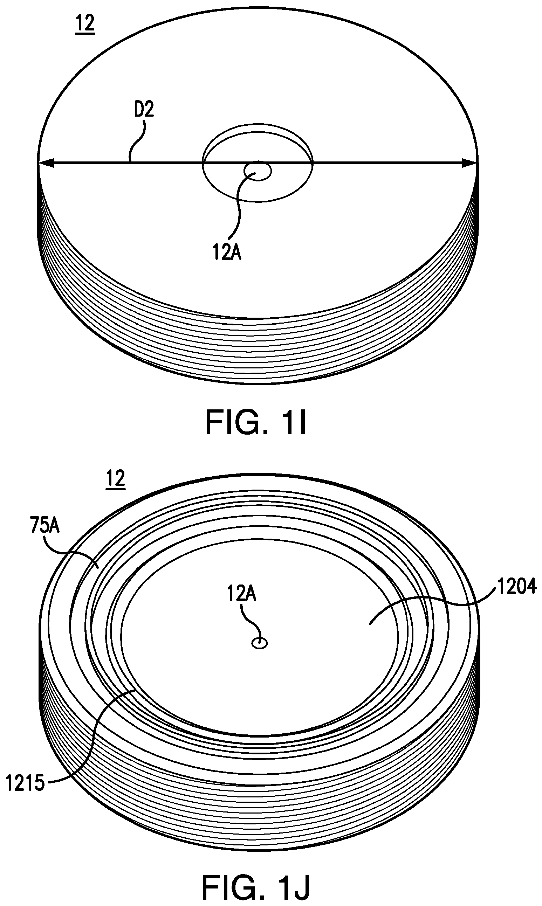

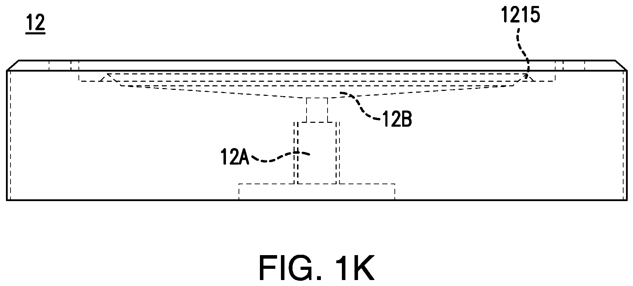

Figures 1A-1K are drawings showing an aspect of a chromatography device according to an aspect of the invention, having a planar filter configuration. Figures 1A and 1B show, respectively, top and bottom perspective views of an assembled chromatography device having a housing and an annular outlet section engaged with the housing; Figure 1C shows a cross-sectional view of the assembled device shown in Figure 1A, also showing an annular inlet plate and a filter; Figure 1D shows an exploded view of the device, Figures 1F, 1G, and 1H show, respectively, top perspective, bottom perspective, and partial cut-away views of the annular inlet plate; Figures 1I, 1J, and 1K show, respectively, top perspective, bottom perspective, and partial cut-away views of the annular outlet section shown in Figure 1B.

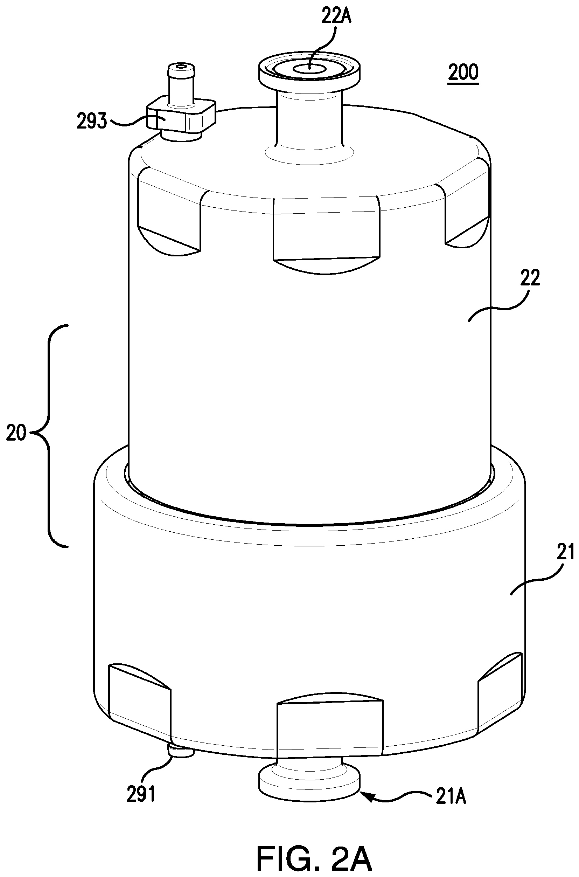

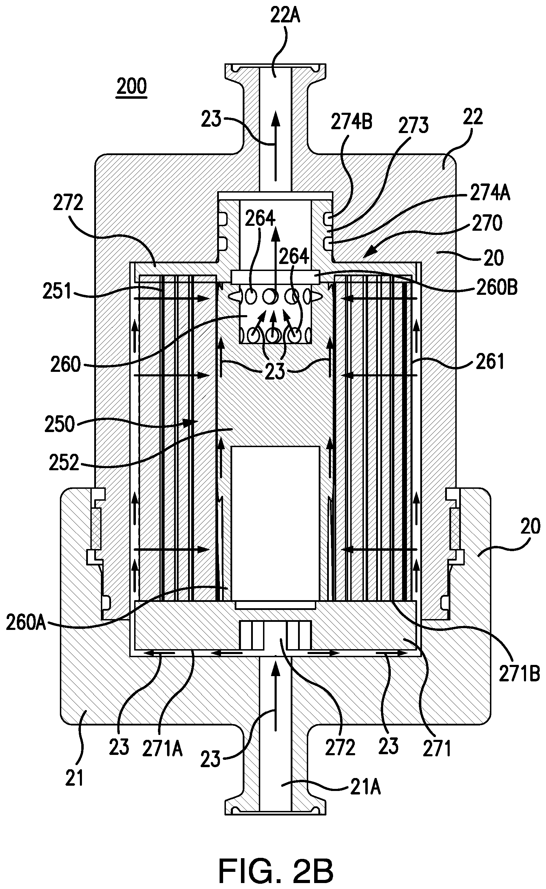

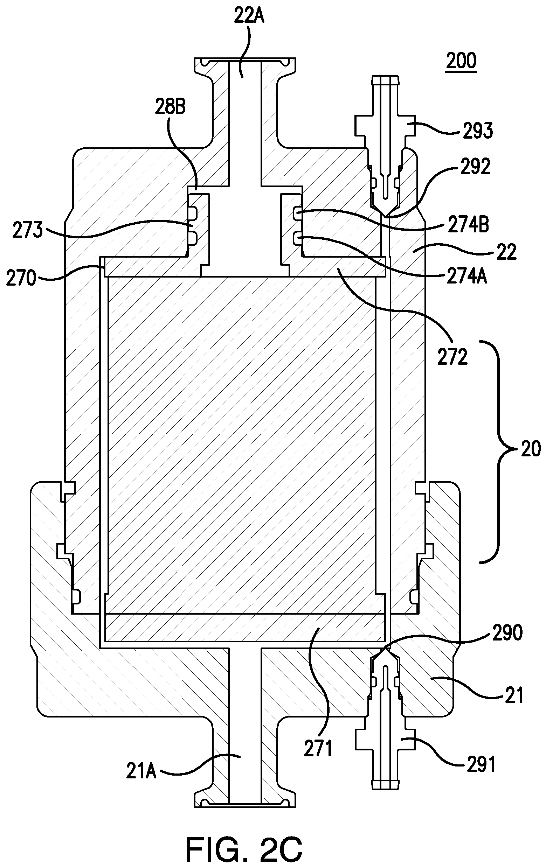

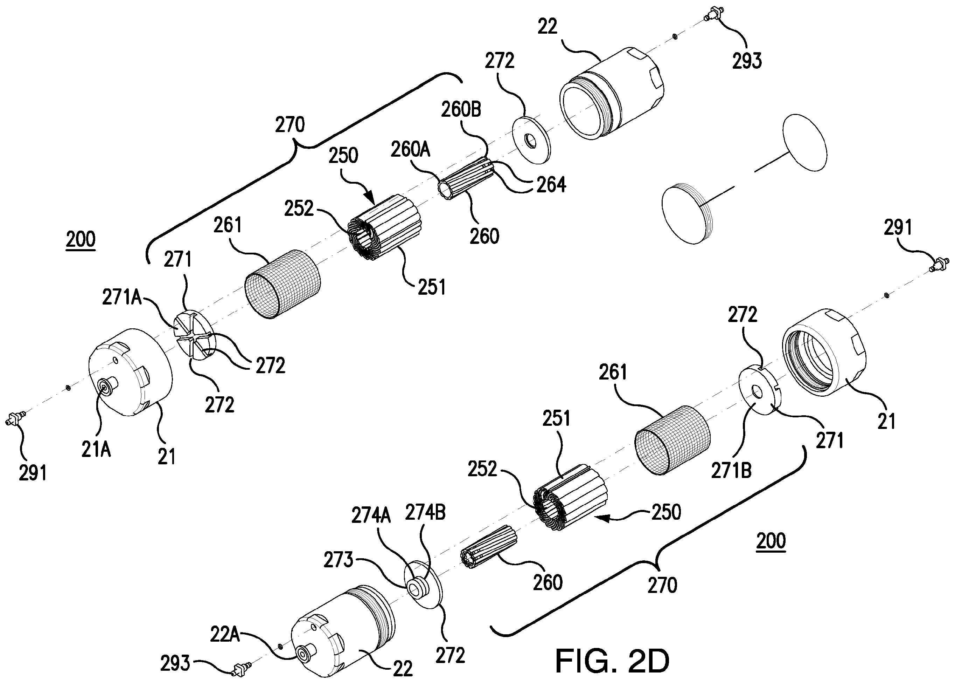

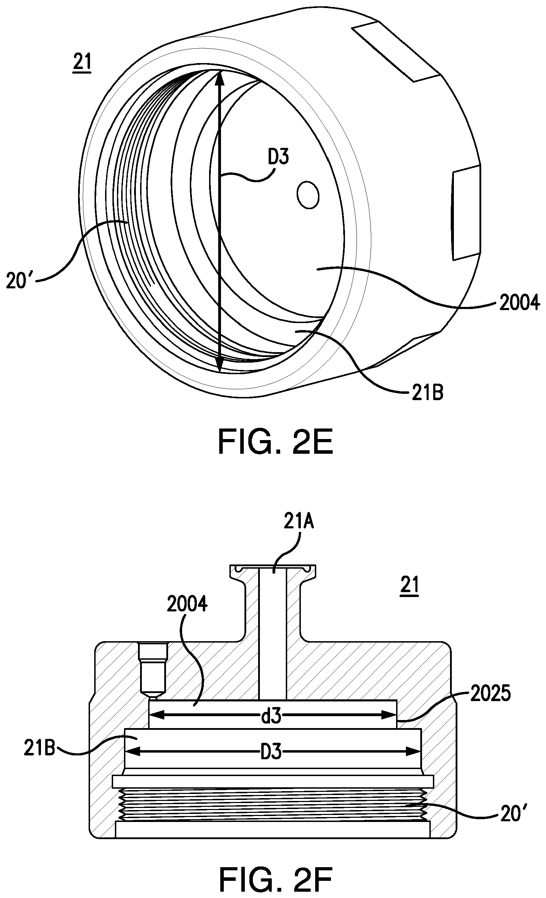

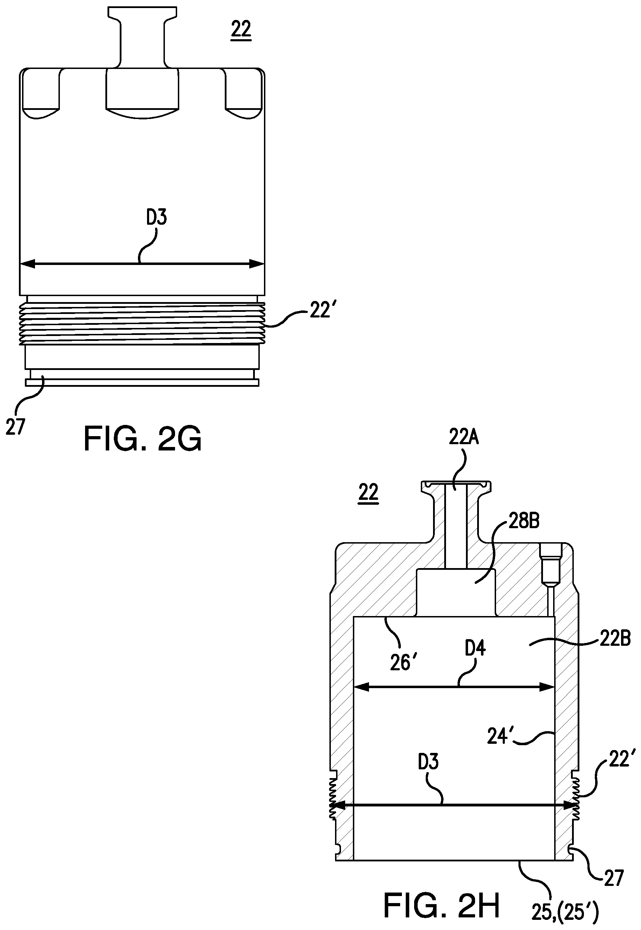

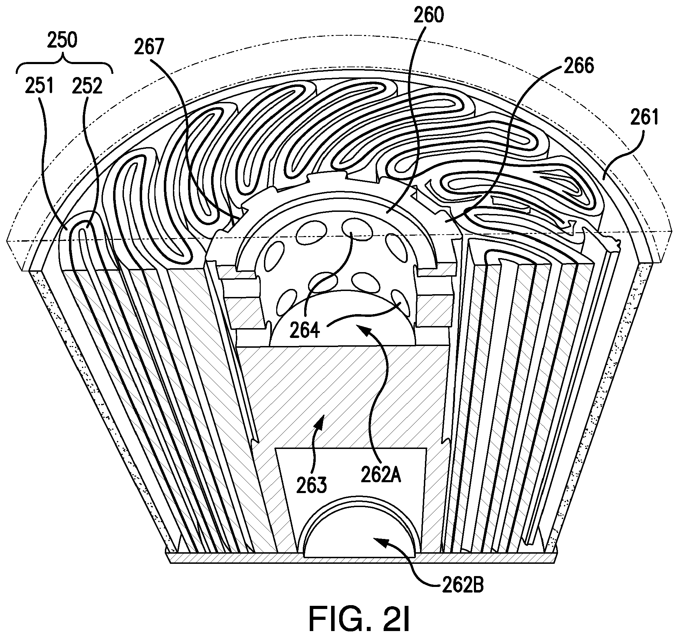

Figures 2A-2I are drawings of a chromatography device according to another aspect of the invention, having cylindrical hollow filter configuration. Figure 2A shows a top perspective view an assembled chromatography device; Figures 2B and 2C show different cross-sectional views of the device shown in Figure 2A; Figure 2D shows front and rear exploded perspective views of the device; Figures 2E and 2F show, respectively, bottom perspective and cross-sectional views of the inlet section of the device, Figures 2G and 2H show, respectively, side and cross-sectional views of the outlet section of the device; and Figure 2I is a drawing showing a top partial cut-away view of the assembled device.

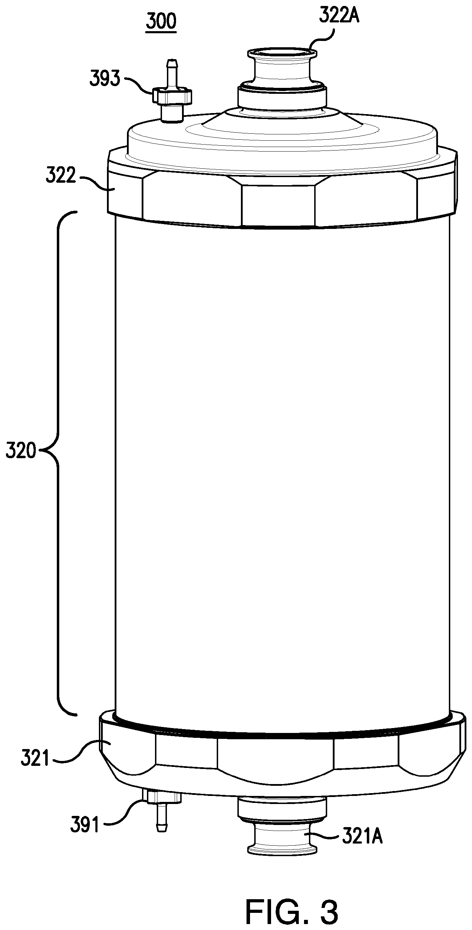

Figure 3 is a drawing showing a perspective view of a chromatography device according to another aspect of the invention.

### DETAILED DESCRIPTION OF THE INVENTION

In accordance with an aspect of the invention, a chromatography device is provided comprising (a) a filter housing having an inlet and an outlet and defining a fluid flow path between the inlet and the outlet; (b) a porous filter arranged in the filter housing across the fluid flow path, the porous filter comprising (i) first porous filter element; and (ii) a second porous filter element in contact with the first porous filter element, the second porous filter element arranged downstream of the first porous filter element, wherein the first porous filter element comprises at least one anionic exchange (AEX) membrane, and the second porous filter element comprises at least one hydrophobic interaction (HIC) membrane.

In one aspect of the chromatography device, the AEX layer(s) and the HIC membrane layer(s) are planar membranes.

In another aspect of the chromatography device, the AEX membrane layer(s) and the HIC membrane layer(s) are arranged to form a hollow cylindrical filter, e.g., to allow radial flow, wherein the AEX membrane surrounds the HIC membrane.

Typical aspects of the chromatography device include at least two AEX membrane layers and at least two HIC membrane layers.

In another aspect, a method for purifying nucleic acidcontaining enveloped viruses, such as lentivirus, is provided, the method comprising (a) passing a fluid comprising enveloped viruses through a chromatography device comprising a filter housing having an inlet and an outlet and defining a fluid flow path between the inlet and the outlet; a porous filter arranged in the filter housing across the fluid flow path, the porous filter comprising first porous filter element; and a second porous filter element in contact with the first porous filter element, the second porous filter element arranged downstream of the first porous filter element, wherein the first porous filter element comprises at least one anionic exchange (AEX) membrane, and the second porous filter element comprises at least one hydrophobic interaction (HIC) membrane; (b) binding the enveloped virus to the at least one AEX membrane; (c) eluting the enveloped viruses from the at least one AEX membraner in high salt; (d) binding the enveloped viruses to the at least one HIC membrane; and, (e) eluting the enveloped viruses from the at least one HIC membrane in low salt buffer.

Advantageously, a single chromatography device can be used, wherein the method for purification is less complex than conventional methods, while reducing buffer consumption. For example, two purification actions are performed in one operation with a high flow rate (10 membrane volumes/min). Lentivirus and AAV can be eluted with low conductivity buffer from hydrophobic interaction media which aids in their stability

Each of the components of the invention will now be described in more detail below, wherein like components have like reference numbers.

Figures 1A-1K are drawings showing an aspect of a chromatography device according to an aspect of the invention, having a planar filter configuration. Figures 1A and 1B show, respectively, top and bottom perspective views of an assembled device having a housing and an annular outlet section engaged with the housing; Figure 1C shows a cross-sectional view of the assembled device shown in Figure 1A, also showing an annular inlet plate and a filter; Figure 1D shows an exploded view of the device, Figures 1F, 1G, and 1H show, respectively, top perspective, bottom perspective, and partial cut-away views of the annular inlet plate; Figures 1I, 1J, and 1K show, respectively, top perspective, bottom perspective, and partial cut-away views of the annular outlet section shown in Figure 1B.

Figures 1A-1K show an aspect of a chromatography device 100 comprising a filter housing 10 having an annular inner diameter D bounded by a threaded inner side wall 10' (for threadable engagement with a threaded outer wall 12' of an annular outlet section 12), the side wall also including an inwardly facing section 1025 having a lesser inner diameter d1 near the bottom surface 1004, the bottom surface 1004 including an annular projecting ring 1015 as well as an annular shoulder 1020 surrounding a base of an aperture 1010 (for receiving a projection of the annular inlet plate); an annular inlet plate 11 having a projection 1101 including an inlet 11A with optional inner threads 1102, an upper surface 1103, and a lower surface 1104 including an annular projecting ring 1115 providing an inlet chamber 11B (shown with a generally concave configuration, wider toward the filter); and an annular outlet section 12A having an annular outer diameter D2 bounded by a threaded outer wall 12', the outlet section including an inner surface 1204 including a groove 75A including a resilient element 71 such as an o-ring and an inner groove 75B, including an annular projecting ring 1215 providing an outlet chamber 12B (shown with a generally concave configuration, wider toward the filter), and an outlet 12A with inner threads 1202, the device defining a fluid flow path 13 between the inlet and the outlet with a porous filter 150 (shown having a planar configuration) arranged in the filter housing across the fluid flow path, the filter comprising a first porous filter element 151 (that may have at least two membranes/ membrane layers) and a second porous filter element 152 (that may have at least two membrane layers) in contact with the first porous filter element, wherein the first porous filter element comprises at least one anionic exchange (AEX) membrane, and the second porous filter element comprises at least one hydrophobic interaction (HIC) membrane, the first porous filter element 151 being arranged upstream of the second porous filter element 152. Preferably, an optional support element 160 such as a mesh or screen is arranged downstream of the porous filter element, e.g., support element 160 contacting the downstream surface of second porous filter element 152. Alternatively, or additionally, a support element, or another support element can be arranged upstream of the porous filter element, e.g., support element contacting the upstream surface of first porous filter element 151.

While this illustrated aspect shows a threaded inlet and outlet (e.g., for connection with conduits having threaded ends or fittings), aspects of the invention are not so limited, and other connections (e.g., triclamp/sanitary flange connections) are within the skill of the art (*see also,* Figures 2A-2H and 3, showing inlets and outlets each having a connection face with an annular groove for receiving a resilient member such as an o-ring).

Figures 2A-2I are drawings showing another aspect of a chromatography device 200 (Figure 2A showing a perspective view, and Figures 2B and 2C showing different cross-sectional views of an assembled device) comprising a filter housing 20 having an inlet section 21 with an inlet 21A, the inlet section having an inlet chamber 21B having an annular inner diameter D3 bounded by a threaded inner side wall 20' (for threadable engagement with a threaded outer wall 22' of outlet section 22), the side wall also including an inwardly facing section 2025 having a lesser inner diameter d3 near the bottom surface 2004; and an outlet section 22 with an outlet 22A, having a lower end 25 with a bottom surface 25' (that will contact the top surface of the inwardly facing section 2025 of the inlet section when the inlet and outlet sections are fully engaged), an annular outer diameter D3 bounded by a threaded outer side wall 22' (for threadable engagement with the threaded inner wall 20' of the inlet section), and an annular inner diameter D4 bounded by an inner side wall 24', providing filter chamber 22B having a top wall 26', and a filter cartridge outlet chamber 28B (above/downstream of the filter chamber 22B) for receiving the outlet 273 for filter cartridge 270. In this illustrated aspect (*see,* Figures 2G and 2H), a no-threaded portion of side wall 22' near the lower end 25 includes a groove 27 for receiving a resilient element such as an o-ring (e.g., for improving a fluid tight seal between the inlet and outlet sections); Figure 2I is a drawing showing a top partial cut-away view of the device

The device 200 defines a fluid flow path 23 (*see,* Figure 2B) between the inlet and the outlet with a filter cartridge 270 including a hollow cylindrical porous filter 250 (*see,* Figure 2I), the filter cartridge and filter being arranged in the filter housing across the fluid flow path, the hollow cylindrical porous filter comprising a first porous filter element 251 and a second porous filter element 252 in contact with the first porous filter element, wherein the first porous filter element comprises at least one anionic exchange (AEX) membrane, and the second porous filter element comprises at least one hydrophobic interaction (HIC) membrane, the first porous filter element 251 being arranged upstream of and surrounding the second porous filter element 252 (each porous filter having a hollow cylindrical configuration; Figure 2I shows the filter 250 with laid-over pleated filter elements 251 and 252) The illustrated filter cartridge has a closed lower end cap 271 (having a lower surface 271A having grooves 272 (for directing flow outwardly) and an upper surface 271B) and an open upper end cap 272 with a hollow extension cartridge outlet 273 (having an outer wall having at least one groove, shown with two grooves 274A, 274B, for receiving resilient elements such as o-rings), and optional support elements such an inner core 260 having a first end 260A and a second end 260B (the ends having respective open chambers 262A, 262B, with a solid section 263 in between) and a side wall with perforations 264 passing through the side wall into the open chamber near the second end 260B (the illustrated inner core has ribs 266 and grooves 267 in the outer surface of the side wall, shown arranged with a continuous angle from the first end to the second end, which can improve channeling liquid toward the outlet), and an outer mesh or cage 261.

In some aspects, the first porous filter element 251 is a pleated element, and can be a laid-over pleat element. In some aspects, the second porous filter element 252 is a pleated element, and can be a laid-over pleat element. For example, the first and second porous elements can both be pleated elements (e.g., as shown in Figure 2I). Alternatively, the second porous element can wound around the first porous element.

Preferably, the device 200 includes vents, wherein inlet section 21 includes threaded vent port 290 and threaded vent valve 291, and outlet section 22 includes threaded vent port 292 and threaded vent valve 293.

In those aspects wherein the device includes vents, the device should be operated in upward flow with the vent(s) open for efficient air displacement from the device. Accordingly, the inlet side of the device should be oriented face down such that air can be displaced from the device in order to establish uniform fluid flow through the filter. Failure to follow this procedure can result in increased pressure and decreased chromatographic performance, and so it may be desirable to connect a pressure gauge upstream of the device inlet and monitor the pressure. The vent(s) should be opened when the liquid flow is initially established, and closed when all of the air is displaced by liquid. The inlet side of the device should then be oriented face up and liquid passed through the device.

Figure 3 is a drawing showing a perspective view of another aspect of a chromatography device 300 according to the invention, comprising a filter housing 320 having an inlet section 321 with an inlet 321A, and outlet section 322 (threadably engageable with the inlet section), with threaded vent valves 391 and 393. With the exception of the lengths of the inlet section 321 and the outlet section 322 (and the corresponding lengths of the internal elements (such as those comprising the filter cartridge, the inlet chamber, the filter chamber and the filter cartridge outlet chamber, etc.)), and the diameter of the connections for the inlet 321A and the outlet 322A (diameters are larger in device 300), the structure and operation of device 300 essentially corresponds to that of device 200.

### Anion Exchange Membrane

In general, the anionic exchange (AEX) membrane is any semi-permeable membrane comprising a base matrix comprising fixed positively charged (cationic) functional groups and mobile anions (counterions) that enable the selective permeability of negatively charged (anionic) species. The base matrix is any suitable material, preferably a hydrophilic material, such as polyether sulfone (PES), cellulose, a methacrylate, a hydrophilic polymer, or any combination thereof.

In some aspects, the base matrix in the AEX membrane comprises a polyether sulfone matrix that is hydrophilic and may or may not include a crosslinked coating. In a preferred aspect of the invention, the polyether sulfone matrix has a crosslinked coating comprising pendant cationic groups, particularly quaternary ammonium groups.

The crosslinked coating typically comprises a polyamine, a diallylamine copolymer (e.g., a diallylamine copolymer with epoxy groups), and/or an acrylic copolymer, in which one or more of these components includes pendant cationic groups attached directly or indirectly to the backbone of the polymer. If necessary, the diallylamine copolymer can be reacted with epichlorohydrin to provide epoxy sites. The polyamine can be, e.g., diethylenetriamine, triethylenetetramine, tertaethylenepentamine, pentaethylenehexamine, or polyethylenimine.

For example, a crosslinked coating can comprise the reaction product of a polyamine (e.g., polyethylenimine) with pendant cationic groups (e.g., quaternary ammonium groups) and optionally a polyalkyleneglycol polyglycidylether. In another example, a crosslinked coating is prepared by crosslinking a composition comprising diallylamine, a diallyldialkylammonium halide, an acrylic monomer having a cationic group (e.g., a quaternary ammonium group), and a crosslinking agent. In another example, a crosslinked coating is prepared by crosslinking a composition comprising a diallylamine copolymer having epoxy groups and pendant cationic groups, a polyethylenimine, and an amine reactive compound having a cationic group (e.g., glycidyl trimethylammonium chloride). In another example, the crosslinked coating comprising a diallylamine copolymer, an acrylic monomer having a quaternary ammonium group, and a crosslinking agent (e.g., an N-(alkoxymethyl)acrylamide). In another example, the crosslinked coating comprises a diallylamine copolymer, an acrylic monomer having a quaternary ammonium group (e.g., an acryloylaminoalkyl trialkylammonium halide or acryloyloxyalkyl trialkylammonium halide), and a crosslinking agent. In another example, the crosslinked coating has pendant cationic groups and comprises an acrylic copolymer comprising a polymerized monomer selected from the group consisting of glycidylalkylacrylate, methacryloyloxyalkyl trialkylammonium halide, and methacryloylaminoalkyl trialkylammonium halide. In another example, the crosslinked coating has pendant cationic groups and comprises an acrylic copolymer linked to pentaethylenehexamine.

The coating can be crosslinked using any suitable method and/or crosslinking agent. The crosslinking agent is a polyfunctional agent with functional groups that are reactive with a group (e.g., an amino) in the backbone of one more of the polymers in the coating, such as urea, halo, epoxy, isocyanato, carboxyl, or acid chloride. A preferred crosslinking agent is a polyglycidyl compound. An example of a suitable polyglycidyl compound is a polyalkyleneglycol polyglycidylether. Examples of a crosslinking agent include ethyleneglycol diglycidyl ether, 1,4-butanediol diglycidyl ether, N-(isobutoxymethyl)acrylamide, N-(isobutoxymethyl)methacrylamide, epichlorohydrin, epibromohydrin, and a combination thereof. The crosslinking can take place in a solvent comprising water, at least one organic solvent, or a mixture thereof. If necessary, a catalyst can be used, such as a strong base (e.g., sodium hydroxide, potassium hydroxide).

In a preferred aspect, the coating solution comprises a copolymer of a diallylamine, diallyldialkylammonium halide, an acrylic monomer having a cationic group (e.g., quaternary ammonium group), and a crosslinking agent.

The coating composition can be prepared, e.g., by dissolving the monomers (e.g., polyamine) in a suitable solvent (e.g., water, alcohol, such as methanol, ethanol, and propanol, and combinations thereof). The solvent can be present in an amount of from about 40% to about 99%, and preferably in an amount of from about 90% to about 99% by weight of the coating composition. The monomers can be present in an amount of from about 1% to about 5%, and preferably in an amount of from about 1% to about 2.5% by weight of the coating composition. Once the coating solution is applied to the base matrix using any suitable technique (e.g., dipping, spraying, etc.), the solution can be cured to form the crosslinked coating on the base matrix, such that an AEX membrane is formed.

A PES base matrix comprising a crosslinked coating is further described in, for example, U.S. Patent 6,780,327.

In some aspects, the base matrix in the AEX membrane comprises cellulose. Suitable cellulosic polymers include cellulose, regenerated cellulose, cellulose ester (e.g., cellulose acetate, cellulose propionate, cellulose butyrate, cellulose acetate butyrate), cellulose nitrate (also known as nitrocellulose), methylcellulose, ethylcellulose, and combinations thereof (e.g., a mixture of cellulose acetate and cellulose nitrate).

In some aspects, the base matrix in the AEX membrane comprises a methacrylate polymer. Examples of a methacrylate polymer include polymethyl methacrylate and methyl methacrylate copolymer.

In some aspects, the base matrix in the AEX membrane comprises a hydrophilic polymer. Examples of suitable hydrophilic polymers include a polyamide, a polyimide, a polyalkylene oxide, hydrophilic polyurethane, polyvinylpyrrolidone, polyacrylamide, polyacrylic acid, and polyvinyl alcohol. Examples of a polyamide include a nylon, such as nylon-4,6, nylon-6, nylon-6,6, nylon-11, and/or nylon-12. Examples of a polyimide include an aliphatic polyimide, an aromatic polyimide, or a mixture thereof, such as polyimide derived from poly(amic acid) salt precursor. Examples of a polyalkylene oxide include C₁-C₁₀ polyalkylene oxide, such as polymethylene oxide, polyethylene oxide, polypropylene oxide, and polybutylene oxide. Alternatively, the hydrophilic polymer can be a hydrophobic polymer modified to have hydrophilic functional groups (e.g., hydroxy, amino, aldehyde, carboxy, thio, phosphato, etc.). Such hydrophobic polymers include polyolefins (polyethylene, polypropylene), polystyrene, polycarbonates, fluoropolymers (e.g., polyvinylidene fluoride, polytetrafluoroethylene), and polyether ether ketone (PEEK).

The pendant cationic functional groups are fixed (e.g., covalently bound) to the base matrix (or a crosslinked coating thereon) and comprise any suitable cationic group, such as ammonium, sulfonium, phosphonium, or a combination thereof. In some embodiments, the cationic functional groups are quaternary ammonium groups, in which the nitrogen is bound to the matrix and three other moieties. In general, the quaternary ammonium group has the structure -NR₃⁺, in which each instance of R is the same or different and is hydrogen, C₁₋₁₀ alkyl, C₃₋₈ cycloalkyl, or aryl. Preferably, the quaternary ammonium group is a trialkylammonium.

The counteranion of the cationic functional group is not limited, as the counteranion is mobile and does not affect the permeability of the sample to be processed. For example, the counteranion can be hydride, halide (e.g., F⁻, Cl⁻, Br⁻, or I⁻), azide, carbonate, hydrogen carbonate, chlorate, hypochlorite, perchlorate, hydroxide, cyanide, phosphate, sulfate, sulfite, sulfonate, thiosulfate, acetate, formate, oxalate, or a combination thereof. In a preferred aspect, the counteranion is a halide.

In some embodiments, the fixed cationic functional group can comprise primary amino groups, secondary amino groups, or a combination thereof, which can form a cationic group upon use of the chromatography device. These groups can be the only pendant functional groups or in addition to pendant cationic functional groups.

The pendant cationic functional group are directly or indirectly bound to the base matrix. Directly bound means a bond exists between the base matrix (or a crosslinked coating thereon) and the pendant cationic functional group. Indirectly bound means a spacer is present between the base matrix (or a crosslinked coating thereon) and the pendant cationic functional group. The spacer can be of any suitable length or structure (e.g.., linear or branched) and typically will include one or more alkylene (-CH₂-) groups and optionally one or more functional groups selected from hydroxy, alkoxy, amino, alkylamino, amido, ester, urea, urethane, and a combination thereof. The spacer group can of any suitable length. For example, the spacer group can be a group having from 1 to 10 (e.g., 1, 2, 3, 4, 5, 6, 7, 8, 9, or 10) atoms (e.g., carbon atoms). In an aspect, the spacer group can be from 2 to about 6 (i.e., 1, 2, 3, 4, 5, or 6) atoms long, and preferably 3 carbon atoms long.

In some aspects, the base matrix (or a crosslinked coating thereon) is polymerized in which at least one of the monomers includes the cationic functional group, such that, when formed, the base matrix already includes the cationic functional group. In other aspects, the base matrix (or a crosslinked coating thereon) is modified after its initial synthesis to add one or more cationic functional groups that are either directly or indirectly bonded, as described herein. In an example, the base matrix comprises pendant amino groups that are quaternized to form pendant ammonium groups.

The AEX membrane can have any suitable pore structure, e.g., a pore size (for example, as evidenced by bubble point, or by KL as described in, for example, U.S. Patent 4,340,479, or evidenced by capillary condensation flow porometry), a mean flow pore (MFP) size (e.g., when characterized using a porometer, for example, a Porvair Porometer (Porvair plc, Norfolk, UK), or a porometer available under the trademark POROLUX (Porometer.com; Belgium)), a pore rating, a pore diameter (e.g., when characterized using the modified OSU F2 test as described in, for example, U.S. Patent 4,925,572), or removal rating media. The pore structure used depends on the size of the particles to be utilized, the composition of the fluid to be treated, and the desired effluent level of the treated fluid.

For example, the AEX membrane can have, for example, an average pore diameter of from about 0.01 µm to about 10 µm. For example, the average pore size is 0.01 µm or more (e.g., 0.01 µm or more, 0.02 µm or more, 0.05 µm or more, 0.1 µm or more, 0.2 µm or more, 0.3 µm or more, 0.5 µm or more, 0.8 µm or more, 1 µm or more, 2 µm or more, or 5 µm or more). Alternatively or in combination, the average pore size is 10 µm or less (e.g., 8 µm or less, 5 µm or less, 2 µm or less, 1 µm or less, 0.8 µm or less, 0.5 µm or less, 0.3 µm or less, 0.2 µm or less, 0.1 µm or less, 0.08 µm or less, 0.05 µm or less, or 0.02 µm or less). Any two of the foregoing endpoints can be used to define a close-ended range, or a single endpoint can be used to define an open-ended range. In some aspects, the average pore sizes is from about 0.1 µm to about 5 µm, about 0.1 µm to about 2 µm, 0.2 µm to about 5 µm, 0.2 µm to about 1 µm, about 0.1 µm to about 0.2 µm, about 0.5 µm to about 2 µm, about 0.05 µm to about 0.3 µm, 0.1 µm, 2 µm, or 0.45 µm.

The AEX membrane can have any suitable thickness, such as about 50 µm to about 300 µm. For example, the average thickness can be 50 µm or more (e.g., 60 µm or more, 70 µm or more, 80 µm or more, 100 µm or more, 120 µm or more, 140 µm or more, 160 µm or more, 180 µm or more, 200 µm or more, 220 µm or more, 240 µm or more, 260 µm or more, or 280 µm or more). Alternatively or in combination, the average thickness is 300 µm or less (e.g., 280 µm or less, 260 µm or less, 240 µm or less, 220 µm or less, 200 µm or less, 180 µm or less, 160 µm or less, 140 µm or less, 120 µm or less, 100 µm or less, 80 µm or less, 70 µm or less, or 60 µm or less). Any two of the foregoing endpoints can be used to define a close-ended range, or a single endpoint can be used to define an open-ended range. In some aspects, the average pore size is from about 60 µm to about 200 µm, about 80 µm to about 170 µm, 90 µm to about 160 µm, 100 µm to about 200 µm, or about 100 µm to about 200 µm.

### Hydrophobic Interaction Membrane

In general, the hydrophobic interaction (HIC) membrane is any semi-permeable membrane comprising a base matrix comprising fixed alkyl- and/or phenyl-containing groups. The base matrix is any suitable material, such as polyether sulfone (PES), cellulose, a methacrylate, a hydrophilic polymer, or any combination thereof.

In some aspects, the base matrix of the HIC membrane comprises a polyether sulfone matrix that may or may not include a crosslinked coating. The crosslinked coating can be the same as described with respect to the AEX membrane, but in which one or more monomers includes an alkyl- and/or phenyl-containing group or the crosslinked coating is later modified to include alkyl- and/or phenyl-containing groups.

In some aspects, the base matrix of the HIC membrane comprises cellulose. Suitable cellulosic polymers include cellulose, regenerated cellulose, cellulose ester (e.g., cellulose acetate, cellulose propionate, cellulose butyrate, cellulose acetate butyrate), hydrolyzed cellulose ester, cellulose nitrate (also known as nitrocellulose), methylcellulose, ethylcellulose, and combinations thereof (e.g., a mixture of cellulose acetate and cellulose nitrate).

In a preferred aspect of the invention, the HIC membrane comprises hydrolyzed cellulose acetate comprising fixed alkyl- and/or phenyl-containing groups, preferably phenyl groups. In some embodiments of this aspect, the cellulose acetate has been reinforced with, for example, fiber comprising cellulose (e.g., regenerated cellulose).

In an example, the base matrix comprises cellulose ester (e.g., cellulose acetate) that is contacted with a solution that swells the cellulose ester matrix and/or hydrolyzes the ester groups to hydroxyl groups to form a cellulose hydrate (hydrolyzed cellulose acetate) matrix. The solution comprises water, one or more organic solvents, such as an alcohol (e.g., ethanol), or a combination thereof. To simultaneously swell and hydrolyze the cellulose ester, the solution requires water and optionally an organic solvent. Otherwise, the swelling and hydrolysis can be performed stepwise. The solution can further comprise one or more additives, such as an acid (e.g., acetic acid), a salt of an acid (e.g., a Group I or II salt of chloride, sulfate, nitrate, phosphate, acetate, formate, etc., such as sodium chloride, sodium sulfate, sodium acetate, etc.), and a combination thereof. If desired, the cellulose hydrate matrix can be crosslinked with one or more crosslinking agents, solvents, and/or catalysts, as described herein.

In some aspects, the base matrix of the HIC membrane comprises a methacrylate polymer. Examples of a methacrylate polymer include polymethyl methacrylate and methyl methacrylate copolymer.

In some aspects, the base matrix of the HIC membrane comprises a hydrophilic polymer. Examples of suitable hydrophilic polymers include a polyamide, a polyimide, a polyalkylene oxide, hydrophilic polyurethane, polyvinylpyrrolidone, polyacrylamide, polyacrylic acid, and polyvinyl alcohol. Examples of a polyamide include a nylon, such as nylon-4,6, nylon-6, nylon-6,6, nylon-11, and/or nylon-12. Examples of a polyimide include an aliphatic polyimide, an aromatic polyimide, or a mixture thereof, such as polyimide derived from poly(amic acid) salt precursor. Examples of a polyalkylene oxide include C₁-C₁₀ polyalkylene oxide, such as polymethylene oxide, polyethylene oxide, polypropylene oxide, and polybutylene oxide.

The pendant alkyl- and/or phenyl-containing groups are fixed (e.g., covalently bound) to the base matrix (or a crosslinked coating thereon).

In general, the alkyl portion of the alkyl-containing group is a C₁-₁₀ alkyl (e.g., C₁, C₂, C₃, C₄, C₅, C₆, C₇, C₈, C₉, or C₁₀ alkyl) that is linear or branched. Examples of the alkyl group include methyl, ethyl, n-propyl, n-butyl, s-butyl, i-butyl, t-butyl, n-pentyl, i-pentyl, neo-pentyl, t-pentyl, hexyl, heptyl, octyl, nonyl, and decyl. The alkyl-containing group can be unsubstituted or substituted with one or more (e.g., 1, 2, 3, 4, 5, or 6) substituents, such as C₃₋₈ cycloalkyl (C₃, C₄, C₅, C₆, C₇, or C₈ cycloalkyl), halo (e.g., F, Br, Cl, and/or I), halo-C₁-C₁₀ alkyl, nitro, C₂-₆ alkenyl, aryl (e.g., phenyl, naphthyl), and a combination thereof. The substituents can be the same or different, but preferably they are the same. In some embodiments, the alkyl-containing group is n-butyl, hexyl, or octyl.

In general, the phenyl portion of the phenyl-containing group is unsubstituted or substituted with one or more (e.g., 1, 2, 3, 4, 5, or 6) substituents, such as C₁-₁₀ alkyl (e.g., C₁, C₂, C₃, C₄, C₅, C₆, C₇, C₈, C₉, or C₁₀ alkyl), C₃₋₈ cycloalkyl (C₃, C₄, C₅, C₆, C₇, or C₈ cycloalkyl), halo (e.g., F, Br, Cl, and/or I), nitro, C₂-₆ alkenyl, aryl (e.g., phenyl, naphthyl), and a combination thereof. The substituents can be the same or different, but preferably they are the same. When the phenyl is substituted with a substituent, the aromatic ring hydrogen is replaced with the substituent and this can take place at any of the available hydrogens, e.g., 2-, 3-, 4-, 5-, and/or 6-position wherein the 1-position is the point of attachment of the phenyl group to the base matrix, either directly or indirectly. In some embodiments, the phenyl-containing group is phenyl.

In any of the aspects above, whenever a range of the number of atoms in a structure is indicated (e.g., a C₁₋₁₀, C₁₋₈, C₁₋₆, C₁₋₄, etc.), it is specifically contemplated that any sub-range or individual number of carbon atoms falling within the indicated range also can be used. Thus, for instance, the recitation of a range of 1-8 carbon atoms (e.g., C₁-C₈), 1-6 carbon atoms (e.g., C₁-C₆), 1-4 carbon atoms (e.g., C₁-C₄), 1-3 carbon atoms (e.g., C₁-C₃), or 2-8 carbon atoms (e.g., C₂-C₈) as used with respect to any chemical group (e.g., alkyl, cycloalkyl, etc.) referenced herein encompasses and specifically describes 1, 2, 3, 4, 5, 6, 7, and/or 8 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 1-7 carbon atoms, 1-8 carbon atoms, 2-3 carbon atoms, 2-4 carbon atoms, 2-5 carbon atoms, 2-6 carbon atoms, 2-7 carbon atoms, 2-8 carbon atoms, 3-4 carbon atoms, 3-5 carbon atoms, 3-6 carbon atoms, 3-7 carbon atoms, 3-8 carbon atoms, 4-5 carbon atoms, 4-6 carbon atoms, 4-7 carbon atoms, 4-8 carbon atoms, etc., as appropriate).

The pendant alkyl- and/or phenyl-containing groups are directly or indirectly bound to the base matrix (or a crosslinked coating thereon). Directly bound means a bond exists between the base matrix (or a crosslinked coating thereon) and the pendant alkyl- and/or phenyl-containing group. Indirectly bound means a spacer is present between the base matrix (or a crosslinked coating thereon) and the pendant alkyl- and/or phenyl-containing group. The spacer can be of any suitable length or structure (e.g.., linear or branched) and typically will include one or more alkylene (-CH₂-) groups and optionally one or more functional groups, such as hydroxy, halo, alkoxy, amino, alkylamino, amido, ester, urea, urethane, and a combination thereof. The spacer group can of any suitable length. For example, the spacer group can be a group having from 1 to 10 (e.g., 1, 2, 3, 4, 5, 6, 7, 8, 9, or 10) atoms (e.g., carbon atoms). In an aspect, the spacer group can be from 2 to about 6 (i.e., 1, 2, 3, 4, 5, or 6) atoms long, and preferably 3 carbon atoms long.

In some aspects, the base matrix (or a crosslinked coating thereon) is polymerized in which at least one of the monomers includes the alkyl- and/or phenyl-containing group, such that, when formed, the base matrix (or a crosslinked coating thereon) already includes the alkyl- and/or phenyl-containing group. In other aspects, the base matrix (or a crosslinked coating thereon) is modified after its initial synthesis to add one or more alkyl- and/or phenyl-containing groups that are either directly or indirectly bonded, as described herein. In an example, the base matrix comprises one or more functional groups, such as hydroxyl, epoxide, and/or aldehyde groups, that can be modified to add the alkyl- and/or phenyl-containing group using, for example, alkyl glycidyl ether, alkylamine (e.g., butylamine, hexylamine, octylamine), phenyl glycidyl ether, or aniline.

The HIC membrane can have any suitable pore structure, e.g., a pore size (for example, as evidenced by bubble point, or by KL as described in, for example, U.S. Patent 4,340,479, or evidenced by capillary condensation flow porometry), a mean flow pore (MFP) size (e.g., when characterized using a porometer, for example, a Porvair Porometer (Porvair plc, Norfolk, UK), or a porometer available under the trademark POROLUX (Porometer.com; Belgium)), a pore rating, a pore diameter (e.g., when characterized using the modified OSU F2 test as described in, for example, U.S. Patent 4,925,572), or removal rating media. The pore structure used depends on the size of the particles to be utilized, the composition of the fluid to be treated, and the desired effluent level of the treated fluid.

For example, the HIC membrane can have for example, an average pore diameter of from about 0.1 µm to about 20 µm. For example, the average pore size is 0.1 µm or more (e.g., 0.2 µm or more, 0.3 µm or more, 0.5 µm or more, 0.8 µm or more, 1 µm or more, 2 µm or more, 3 µm or more, 5 µm or more, 8 µm or more, 10 µm or more, 12 µm or more, 15 µm or more, or 18 µm or more). Alternatively or in combination, the average pore size is 20 µm or less (e.g., 18 µm or less, 15 µm or less, 12 µm or less, 10 µm or less, 8 µm or less, 5 µm or less, 3 µm or less, 2 µm or less, 1 µm or less, 0.8 µm or less, 0.5 µm or less, 0.3 µm or less, or 0.2 µm or less). Any two of the foregoing endpoints can be used to define a close-ended range, or a single endpoint can be used to define an open-ended range. In some aspects, the average pore size is from about 0.1 µm to about 15 µm, about 0.1 µm to about 10 µm, 0.2 µm to about 5 µm, 0.2 µm to about 1 µm, about 1 µm to about 5 µm, about 2 µm to about 4 µm, 3 µm, or 0.45 µm.

The HIC membrane layer can have any suitable thickness, such as about 50 µm to about 400 µm. For example, the average thickness can be 50 µm or more (e.g., 60 µm or more, 70 µm or more, 80 µm or more, 100 µm or more, 120 µm or more, 140 µm or more, 160 µm or more, 180 µm or more, 200 µm or more, 220 µm or more, 240 µm or more, 260 µm or more, 280 µm or more, 300 µm or more, 320 µm or more, 340 µm or more, 360 µm or more, or 380 µm or more). Alternatively or in combination, the average thickness is 400 µm or less (e.g., 380 µm or less, 360 µm or less, 340 µm or less, 320 µm or less, 300 µm or less, 280 µm or less, 260 µm or less, 240 µm or less, 220 µm or less, 200 µm or less, 180 µm or less, 160 µm or less, 140 µm or less, 120 µm or less, 100 µm or less, 80 µm or less, 70 µm or less, or 60 µm or less). Any two of the foregoing endpoints can be used to define a close-ended range, or a single endpoint can be used to define an open-ended range. In some aspects, the average pore size is from about 60 µm to about 300 µm, about 80 µm to about 270 µm, 90 µm to about 260 µm, 100 µm to about 250 µm, or about 250 µm.

### Both AEX and HIC Membrane Layers

The AEX and HIC membranes can be provided by any suitable method, such as purchased commercially or synthesized using methods known in the art. For example, commercial AEX membranes include the MUSTANG^{™} Q series (Pall, Port Washington NY) and SARTOBIND Q^{™} or SARTOBIND STIC^{™} (Satorius AG, Göttingen, Germany). Commercial HIC membranes include SARTOBIND^{™} Phenyl (Sartorius AG, Göttingen, Germany).

The hydrophilicity and hydrophobicity of the polymers forming the base matrix or the membrane layers can be measured, if necessary, by any suitable method. For example, a drop of water can be can be placed on a film or pressed pellet of the polymer, and the contact angle (i.e., the angle of the liquid meeting the solid surface) of the side view of the water droplet can be measured using, for example, a contact angle goniometer. The sessile drop measurement is the most common method of measuring the contact angle. In general, a contact angle less than 90° is considered a hydrophilic material, whereas a contact angle greater than 90° is considered a hydrophobic material.

Alternatively, the membrane layers can have any desired critical wetting surface tension (CWST, as defined in, for example, U.S. Patent 4,925,572). The porous membrane can have any desired critical wetting surface tension (CWST, as defined in, for example, U.S. Patent 4,925,572). The CWST can be selected as is known in the art, e.g., as additionally disclosed in, for example, U.S. Patents 5,152,905, 5,443,743, 5,472,621, and 6,074,869.

### The Chromatography Device

In a preferred aspect, the chromatography device 100, 200 comprises a porous filter 150 (planar configuration), 250 (radial configuration) comprising an upstream first porous filter membrane 151, 251 comprising at least one AEX membrane comprising a polyether sulfone matrix with a crosslinked coating comprising pendant quaternary ammonium groups, and a downstream second porous filter membrane 152, 252 comprising at least one HIC membrane comprising crosslinked cellulose hydrate comprising pendant phenyl groups.

Chromatography devices according to aspects of the invention can have any number of AEX membrane layers and any number of HIC membrane layers. Typically, the devices have at least two AEX membrane layers and two HIC membrane layers; in some aspects, having, for example, 3, 4, 5, 6, 7, 8, 9, or more, AEX membrane layers and 3, 4, 5, 6, 7, 8, 9, or more, HIC membrane layers. Chromatography devices according to aspects of the invention can have different numbers of AEX membrane layers compared to HIC membrane layers, e.g., a greater number of AEX membrane layers than HIC membrane layers, or vice versa.

The chromatography device can include additional elements, membrane layers, or components, that can have different structures and/or functions, e.g., at least one of any one or more of the following: prefiltration, support, drainage, spacing and cushioning. Illustratively, the filter can also include at least one additional element such as a mesh and/or a screen. In a preferred aspect, the device includes a downstream support element 160, 260.

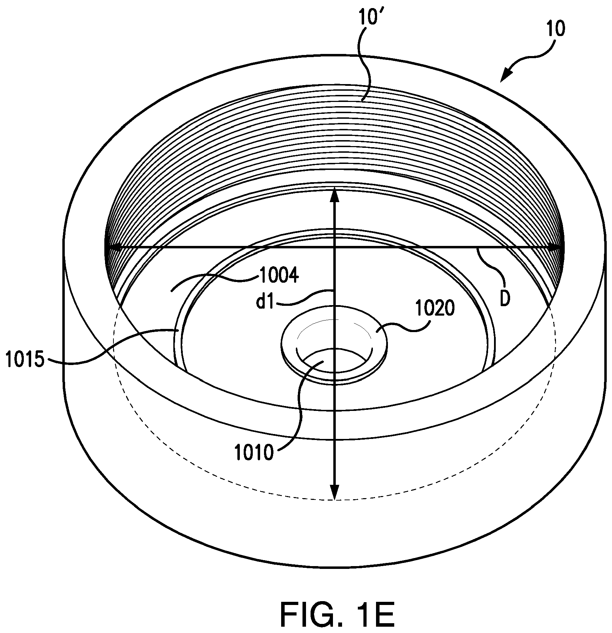

In accordance with aspects of the invention, the filter and filter elements can have a variety of configurations, including planar (e.g., as shown in Figure 1), pleated, and hollow cylindrical (e.g., as shown in Figures 2A, 2B, and 2I).

The chromatography device can be sterilizable. Any filter housing of suitable shape and providing at least one inlet and at least one outlet may be employed. The filter housing can be fabricated from any suitable rigid impervious material, including any impervious thermoplastic material, which is compatible with the fluid being processed. In a preferred aspect, the housing is fabricated from a polymer, such as an acrylic, polypropylene, polystyrene, or a polycarbonated resin.

### Method of Purification

In an aspect, a method of purification comprises (a) passing a fluid comprising nucleic acid-containing enveloped viruses through a chromatography device comprising a filter housing having an inlet and an outlet and defining a fluid flow path between the inlet and the outlet; a porous filter arranged in the filter housing across the fluid flow path, the porous filter comprising a first porous filter element; and a second porous filter element in contact with the first porous filter element, wherein the first porous filter element comprises at least one anionic exchange (AEX) membrane, and the second porous filter element comprises at least one hydrophobic interaction (HIC) membrane; (b) binding the enveloped viruses to the AEX membrane; (c) eluting the enveloped viruses from the AEX membrane in high salt; (d) binding the enveloped virus to the HIC membrane; and, (e) eluting the enveloped virus from the HIC membrane in low salt buffer.

For example, the enveloped virus is captured on the AEX membrane under binding conditions (pH above isoelectric point and conductivity below that required for binding). The membrane is washed with binding buffer and eluted from the AEX membrane with salt solution (such Sodium Citrate or Ammonium Sulfate) and bound onto the HIC membrane. The HIC membrane is washed with salt buffer and eluted with AEX binding buffer (HIC elution buffer).

For purification of enveloped viruses such as lentivirus, the biomolecule of interest is dissolved in buffer having a pH higher than the isoelectric point (thus negatively charged) and conductivity below what is needed for elution from AEX membrane, and passed thorough the chromatography device at 10 membrane volumes/min flow rate, such that the biomolecule binds to the AEX membrane. A wash with binding buffer is executed and the biomolecule is eluted from the AEX membrane using a buffer of high enough conductivity to elute the biomolecule (causing desorption by equilibrating - elution buffer) and to bind the biomolecule to the HIC membrane. The HIC membrane bound with the biomolecule is washed with elution buffer and eluted with a buffer having a pH higher than the isoelectric point of the biomolecule and low conductivity.

Alternative buffers such as, for example, MES, HEPES, MOPS, TAPSO, BES, PIPES, ACES, can be used. Sodium Chloride, Magnesium Chloride at concentration of 1.5 M minimum can be used as alternative to Sodium Citrate or Ammonium Sulfate.

The following examples further illustrate the invention but, of course, should not be construed as in any way limiting its scope.

### EXAMPLE 1

This example demonstrates lentivirus purification using a chromatography device according to an aspect of the invention.

A chromatography device is provided as generally shown in Figure 1C. The chromatography device includes 4 layers of AEX membrane and 4 layers of HIC membrane, minimum 0.1 cm in diameter, fitted into a filter holder with AEX membrane on the inlet (upstream) side and the HIC membrane on the outlet (downstream) side, and is washed with 10 mL of 1 M NaOH solution then equilibrated with 25 mL of 25 mM Tris pH 8, 100 mM NaCl (prepared from 25 mL of 1 M Tris and 25 mL of 5 M NaCl added to 950 mL of DI water).

10 mL of clarified lentivirus harvest from HEK293 cells containing approximately 1.6 x 10⁶ vectors/mL with conductivity of about 15 mS/cm is flown through chromatography device at anywhere from 1 to 10 membrane volumes/min flow rate. The membranes are washed with 4 mL of AEX Equilibration buffer (25mM Tris pH8 100 mM NaC and eluted from AEX media/bound on HIC media with 4 mL of 1.5 M Sodium Citrate pH 8.0 (prepared from adding 441.15 g of Sodium Citrate dihydrate and 25 mL of 1 M Tris pH 8 into in total 1 L of DI water). The device is washed with 4 mL of elution buffer. The HIC membrane is eluted with 4 mL of AEX equilibration buffer (25 mM Tris, pH 8 100 mM NaCl) (a low salt buffer) to contain approximately 8.3 x 10⁵ Vectors/mL free of DNA and host-cell protein (a 15% yield measured by functional titer assay after two cycles of freeze and thaw). The conductivity of eluted lentivirus harvest is about 40 mS/cm.

The lentivirus is recovered in high purity, free of host cell proteins and DNA, without requiring buffer dilution.

### EXAMPLE 2

This example demonstrates lentivirus purification using a chromatography device according to an aspect of the invention, wherein, in contrast with Example 1, the yield is measured without freeze and thaw.

The procedure summarized in Example 1 is generally followed, except that the yield is measured following the manufacturer's instructions for using the Gyrolab^{®} p24 kit for Lentivirus titer determination (2021; Gyros Protein Technologies).

The yield (using an ELISA assay measuring free surface p24 protein) is over 30%.

### EXAMPLE 3

This example describes a chromatography device according to another aspect of the invention.

A chromatography device is provided as generally shown in Figures 2B and 2I. The chromatography device includes 8 layers of AEX pleated membrane and 8 layers of pleated HIC membrane, with the AEX membrane wound around the HIC membrane, fitted into the housing with AEX membrane on the inlet (upstream) side.

In one aspect, the eluted lentivirus is analyzed to determine to lentivirus recovery (Vectors/mL) by Transduction Units (TU) assay using Fluorescence-Activated Cell Sorting (FACS) after freeze and thaw free of DNA and host-cell protein, without requiring buffer dilution.

## Claims

1. A chromatography device for purification of enveloped viruses, such as lentivirus, comprising:
(a) a filter housing having an inlet and an outlet and defining a fluid flow path between the inlet and the outlet;
(b) a porous filter arranged in the filter housing across the fluid flow path, the porous filter comprising:
(i) a first porous filter element; and
(ii) a second porous filter element in contact with the first filter element,
the second porous filter element arranged downstream of the first porous filter element, wherein the first filter element comprises at least one anionic exchange (AEX) membrane, and the second filter element comprises at least one hydrophobic interaction (HIC) membrane.

2. The chromatography device of claim 1, wherein the AEX membrane and the HIC membrane are planar membranes.

3. The chromatography device of claim 1, wherein the porous filter comprises a hollow cylindrical filter wherein the AEX membrane surrounds the HIC membrane.

4. The chromatography device of any one of claims 1 3, wherein the porous filter includes two or more AEX membranes and two or more HIC membranes.

5. A method for purifying enveloped viruses, such as lentivirus, the method comprising:
(a) passing a fluid comprising enveloped viruses through a chromatography device comprising a filter housing having an inlet and an outlet and defining a fluid flow path between the inlet and the outlet; a porous filter arranged in the filter housing across the fluid flow path, the porous filter comprising a first porous filter element; and a second porous filter element in contact with the first filter element, the second porous filter element arranged downstream of the first porous filter element, wherein the first filter element comprises at least one anionic exchange (AEX) membrane, and the second filter element comprises at least one hydrophobic interaction (HIC) membrane;
(b) binding the enveloped viruses to the at least one AEX membrane;
(c) eluting the enveloped viruses from the at least one AEX membrane in high salt;
(d) binding the enveloped viruses to the at least one HIC membrane; and,
(e) eluting the enveloped viruses from the at least one HIC membrane in low salt buffer.

## Patentansprüche

1. Chromatographievorrichtung zur Reinigung von umhüllten Viren, wie beispielsweise einem Lentivirus, umfassend:
(a) ein Filtergehäuse, das einen Einlass und einen Auslass aufweist, und das einen Fluidströmungsweg zwischen dem Einlass und dem Auslass definiert;
(b) einen porösen Filter, der im Filtergehäuse quer zum Fluidströmungsweg angeordnet ist, wobei der poröse Filter Folgendes umfasst:
(i) ein erstes poröses Filterelement; und
(ii) ein zweites poröses Filterelement, das mit dem ersten Filterelement in Kontakt steht,
wobei das zweite poröse Filterelement stromabwärts des ersten porösen Filterelements angeordnet ist, wobei das erste Filterelement mindestens eine Anionenaustauschmembran (AEX) umfasst, und das zweite Filterelement mindestens eine Membran für hydrophobe Wechselwirkungen (HIC) umfasst.

2. Chromatographievorrichtung nach Anspruch 1, wobei die AEX-Membran und die HIC-Membran flächige Membranen sind.

3. Chromatographievorrichtung nach Anspruch 1, wobei der poröse Filter einen hohlzylindrischen Filter umfasst, wobei die AEX-Membran die HIC-Membran umgibt.

4. Chromatographievorrichtung nach einem der Ansprüche 1 bis 3, wobei der poröse Filter zwei oder mehr AEX-Membranen und zwei oder mehr HIC-Membranen einschließt.

5. Verfahren zur Reinigung von umhüllten Viren, wie beispielsweise einem Lentivirus, wobei das Verfahren umfasst:
(a) Leiten eines Fluids, umfassend umhüllte Viren, durch eine Chromatographievorrichtung, umfassend ein Filtergehäusedas einen Einlass und einen Auslass aufweist, und das einen Fluidströmungsweg zwischen dem Einlass und dem Auslass definiert; einen porösen Filter, der im Filtergehäuse quer zum Fluidströmungsweg angeordnet ist, wobei der poröse Filter ein erstes poröses Filterelement umfasst; und ein zweites poröses Filterelement, das mit dem ersten Filterelement in Kontakt steht, wobei das zweite poröse Filterelement stromabwärts des ersten porösen Filterelements angeordnet ist, wobei das erste Filterelement mindestens eine Anionenaustauschmembran (AEX) umfasst, und das zweite Filterelement mindestens eine Membran für hydrophobe Wechselwirkungen (HIC) umfasst;
(b) Binden der umhüllten Viren an die mindestens eine AEX-Membran;
(c) Eluieren der umhüllten Viren aus der mindestens einen AEX-Membran in hohem Salzgehalt;
(d) Binden der umhüllten Viren an die mindestens eine HIC-Membran; und
(e) Eluieren der umhüllten Viren aus der mindestens einen HIC-Membran in einem Puffer mit niedrigem Salzgehalt.

## Revendications

1. Dispositif de chromatographie pour la purification de virus enveloppés, tels qu'un lentivirus, comprenant :
(a) un boîtier filtrant présentant une entrée et une sortie et délimitant un canal d'écoulement de fluide entre l'entrée et la sortie ;
(b) un filtre poreux disposé dans le boîtier filtrant à travers le canal d'écoulement de fluide, le filtre poreux comprenant :
(i) un premier élément de filtre poreux ; et
(ii) un deuxième élément de filtre poreux en contact avec le premier élément de filtre,
le deuxième élément de filtre poreux étant disposé en aval du premier élément de filtre poreux, dans lequel le premier élément de filtre comprend au moins une membrane d'échange d'anions (AEX), et le deuxième élément de filtre comprend au moins une membrane d'interaction hydrophobe (HIC).

2. Dispositif de chromatographie selon la revendication 1, dans lequel la membrane AEX et la membrane HIC sont des membranes planes.

3. Dispositif de chromatographie selon la revendication 1, dans lequel le filtre poreux comprend un filtre cylindrique creux dans lequel la membrane AEX entoure la membrane HIC.

4. Dispositif de chromatographie selon l'une quelconque des revendications 1 à 3, dans lequel le filtre poreux inclut deux membranes AEX ou plus et deux membranes HIC ou plus.

5. Procédé de purification de virus enveloppés, tels qu'un lentivirus, le procédé comprend les étapes consistant à :
(a) faire passer un fluide comprenant des virus enveloppés à travers un dispositif de chromatographie comprenant un boîtier filtrant présentant une entrée et une sortie et délimitant un canal d'écoulement de fluide entre l'entrée et la sortie ; un filtre poreux disposé dans le boîtier filtrant à travers le canal d'écoulement de fluide, le filtre poreux comprenant un premier élément de filtre poreux ; et un deuxième élément de filtre poreux en contact avec le premier élément de filtre, le deuxième élément de filtre poreux étant disposé en aval du premier élément de filtre poreux, dans lequel le premier élément de filtre comprend au moins une membrane d'échange d'anions (AEX), et le deuxième élément de filtre comprend au moins une membrane d'interaction hydrophobe (HIC) ;
(b) lier les virus enveloppés à la au moins une membrane AEX ;
(c) éluer les virus enveloppés à partir de la au moins une membrane AEX dans une solution à forte concentration saline ;
(d) lier les virus enveloppés à la au moins une membrane HIC ; et,
(e) éluer les virus enveloppés à partir de la au moins une membrane HIC dans un tampon à faible concentration saline.
